# EUROPÄISCHE PATENTANMELDUNG

(11) **EP 1 990 004 A1**
(43) Veröffentlichungstag der Anmeldung: **12.11.2008**
(21) Anmeldenummer: 08007803.3
(22) Anmeldetag: 23.04.2008
(51) Int. Cl.: A61B 6/00, A61B 6/12, A61B 19/00

(54) **Kalibrieverfahren für eine Röntgendiagnostikeinrichtung und Vorrichtung hierfür**

(30) Priorität: 05.05.2007 DE 102007021183
(71) Anmelder: Ziehm Imaging GmbH, 90451 Nürnberg (DE)
(72) Erfinder: Dehler, Jürgen, 91301 Forchheim (DE)

(57) **Zusammenfassung**

Die Erfindung betrifft ein Kalibrierverfahren für eine Röntgendiagnostikeinrichtung und eine Vorrichtung zur Verwendung bei dem Kalibrierverfahren, wobei die Vorrichtung ein Lageerfassungssystem mit einer Aufnahmeeinheit und ein Röntgenphantom aufweist, das in einer bezüglich des Koordinatensystems des Lageerfassungssystems bekannten Lage und Orientierung in der Nähe der Aufnahmeeinheit angeordnet und an dieser vorzugsweise lösbar gehalten ist.

## Beschreibung

Die Erfindung betrifft ein Kalibrierverfahren für eine Röntgendiagnostikeinrichtung und eine Vorrichtung zur Verwendung bei dem Kalibrierverfahren.

Medizinische Eingriffe an lebenden Objekten werden zunehmend mittels Navigationsunterstützung durchgeführt. Darunter versteht man die mittels eines Lageerfassungssystems unterstützte Führung eines Instrumentes relativ zu einem in Behandlung stehenden Gewebebereichs des Objektes. Von besonderem Interesse ist die Navigation in Bereichen, die sich einer optischen Kontrolle des Operateurs entziehen, weil das Instrument beispielsweise in das Innere des Objektes eingeführt wurde. Hierzu wird die Führung des Instrumentes, beispielsweise eines Katheders, in einem virtuellen 3D-Volumen vorgenommen, welches mittels eines bildgebenden Verfahrens vor oder während der Operation erzeugt wurde. Eine häufige Anwendung ist, mit Hilfe einer Röntgendiagnostikeinrichtung eine Reihe von 2D-Projektionsaufnahmen bekannter Projektionsgeometrie zu erzeugen und aus diesen 2D-Aufnahmen einen 3D-Volumendatensatz zu erzeugen. Der Volumendatensatz wird an ein Navigationssystem übergeben, das über ein Lageerfassungssystem für von diesem erfaßbaren Marken verfügt. Um eine Navigation mit hoher Genauigkeit möglich zu machen, wird das Koordinatensystem des Lageerfassungssystem mit dem Koordinatensystem des 3D-Volumendatensatzes abgeglichen. Dieser Vorgang wird üblicherweise "Registrierung" genannt. Bei der Registrierung wird beispielsweise ein Phantom, das röntgenpositive Marken und vom Lageerfassungssystem erfaßbare Marken in einer festen Raumbeziehung zueinander enthält, verwendet. Zur Verbesserung der Genauigkeit eines rekonstruierten 3D-Datensatzes aus 2D-Röntgenprojektionsaufnahmen sind Verfahren bekannt, die die Abweichungen der Parameter der Projektionsgeometrie von der realen, beispielsweise durch mechanische Verwindungen der Röntgendiagnostikeinrichtung beeinflußten Projektionsgeometrie berücksichtigt. Hierzu wird eine Röntgendiagnostikeinrichtung unter Verwendung spezieller Röntgenphantome "kalibriert". Eine solche Kalibrierung erfolgt in der Regel lediglich vor der Auslieferung, nach einer Reparatur mit Austausch mechanischer Komponenten oder vor Beginn einer Untersuchung.

Aus der DE 102 02 091 A1 ist eine Vorrichtung und ein Verfahren zur Ermittlung einer Koordinatentransformation unter Verwendung eines Phantoms bekannt, an dem röntgenpositive Marken und von einem Lageerfassungssystem erfaßbare Marken in einer festen räumlichen Beziehung zueinander angeordnet sind. Bei einem Scan zur Erzeugung von 2D-Röntgenprojektionsaufnahmen werden die Koordinaten der röntgenpositiven Marken im rekonstruierten 3D-Volumen ermittelt und zum Abgleich an das Lageerfassungs- und Navigationssystem übermittelt.

Aus der deutschen Patentschrift DE 100 47 382 C2 ist ein Röntgenphantom bekannt, das, ebenso wie das Röntgensystem von einem Lageerfassungssystem erfaßbare Marken aufweist. Durch die Lageerfassung der Marken an dem Röntgenphantom und an dem Röntgensystem werden Koordinaten im Koordinatensystem des Lageerfassungssystems gewonnen, aus denen einen Koordinatentransformation zwischen dem Röntgensystem und dem Röntgenphantom bestimmbar sind.

Die bekannten Kalibrierverfahren unter Verwendung eines Lageerfassungssystems weisen den Nachteil auf, daß für die zu ermittelnde Koordinatentransformation sowohl die Lage des Röntgenphantoms als auch die Lage des Röntgenstrahlenempfängers bzw. eines mit dem Röntgenstrahlenempfänger nach dem kinematischen Modell in fester Relation stehenden Teils der Röntgendiagnostikeinrichtung bestimmt werden muß. Durch die Meßungenauigkeiten der wenigstens zwei Messungen ist die Koordinatentransformation mit einem berechenbaren Fehler behaftet. Geht man beispielsweise von der Verwendung eines Lageerfassungssystems mit zwei in fester Relation zueinander stehender Kameras aus, so ist der Fehler der Koordinatentransformation um so größer, je kleiner der Winkel zwischen einem Punkt des Röntgenphantoms bzw. der Röntgendiagnostikeinrichtung und den beiden Eintrittspupillen der Kameras ist. Für ein Lageerfassungssystem mit einer vorgegebenen Aufnahmeeinrichtung ist der Fehler der Koordinatentransformation um so größer, je größer der Abstand zwischen dem Röntgenphantom bzw. der Röntgendiagnostikeinrichtung und der Aufnahmeeinheit des Lageerfassungssystems ist.

Aufgabe der Erfindung ist es, die Genauigkeit der Kalibrierung einer Röntgendiagnostikeinrichtung unter Verwendung eines Lageerfassungssystems und eines Röntgenphantoms zu verbessern.

Die Aufgabe der Erfindung wird dadurch gelöst, daß für die Bestimmung der Lage des Röntgenphantoms im Koordinatensystem der Röntgendiagnostikeinrichtung lediglich die Lage des Röntgenstrahlenempfängers oder eines Teils der Röntgendiagnostikeinrichtung mit dem Lageerfassungssystem erfaßt wird und das Röntgenphantom in einer bekannten Lage und Orientierung zu und in der Nähe von der Aufnahmeeinheit des Lageerfassungssystems angeordnet und an dieser vorzugsweise lösbar gehalten ist. Zur Kalibrierung wird die Aufnahmeeinheit des Lageerfassungssystems mit dem daran angeordneten Röntgenphantom derart im Strahlengang der Röntgendiagnostikeinrichtung positioniert, daß von dem Röntgenphantom wenigstens zwei Röntgenprojektionsaufnahmen angefertigt werden können und die Position des Röntgenstrahlenempfängers oder der Röntgendiagnostikeinrichtung für wenigstens eine Projektionsgeometrie erfaßbar ist.

Die Erfindung wird anhand der Abbildungen näher erläutert.

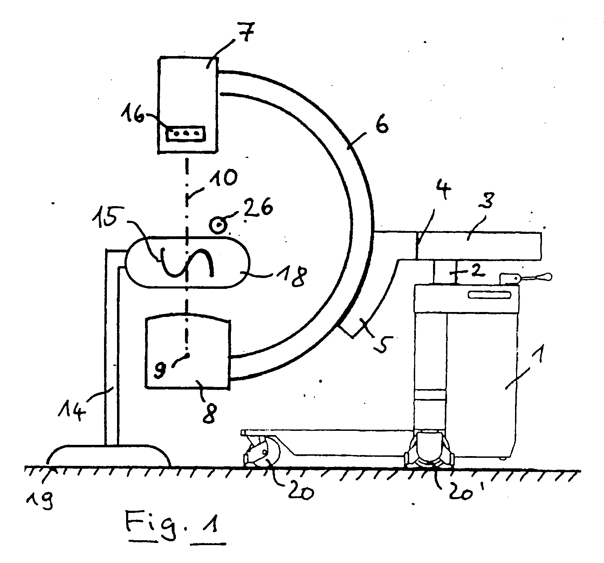

In Fig. 1 ist eine mobile Röntgendiagnostikeinrichtung mit einem auf Rollen (20, 20') längs des Fußbodens (19) verschiebbaren Gerätewagen (1) der einen mehrfach verstellbaren C-Bogen (6) trägt. Die Röntgenstrahlenquelle (8) und der Röntgenstrahlenempfänger (7) sind an den Enden eines C-Bogens (6), der längs seines Umfanges in einer C-Bogenhalterung (5) um den Mittelpunkt (26) verschieblich gelagert ist, angeordnet. Ein Strahlenkegel beziehungsweise eine Strahlenpyramide im Falle eines Röntgenstrahlenempfängers (7) mit einem rechteckigen Eingangsfenster erstreckt sich zwischen dem Brennfleck (9) und dem Eingangsfenster (11) des Röntgenstrahlenempfängers (7). Der Zentralstrahl (10) erstreckt sich von dem Brennfleck (9) zum Mittelpunkt des Eingangsfensters (11) des Röntgenstrahlenempfängers (7). An dem Röntgenstrahlenempfänger (7) ist eine Markenanordnung (16) vorgesehen. Die Position der Markenanordnung (16) kann mittels des Lageerfassungssystems (18) entweder dadurch bestimmt werden, daß die Markenanordnung (16) vom Lageerfassungssystem (18) erfaßbare Marken enthält oder daß die Markenanordnung mit einem Pointer antastbare Punkte aufweist, deren Orientierung durch vorzugsweise mehrfaches Antasten mit dem Pointer im Koordinatensystem des Lageerfassungssystems (18) ermittelbar sind.

In Fig. 1 ist ferner ein Stativ (14) dargestellt, das an seinem oberen Ende die Aufnahmeeinheit des Lageerfassungssystems (18) mit dem Röntgenphantom (15) trägt. Das Röntgenphantom (15) kann aus einer einzigen röntgenpositiven Punktmarke oder einer röntgenpositiven Struktur bestehen. Das Lageerfassungssystem (18) kann ein optisches (Infrarotsystem, Lasermeßsystem, Kamera- bzw. Stereokamerasystem) oder ein auf der Vermessung eines Magnetfeldes oder eines elektrischen Feldes beruhendes System sein.

Die C-Bogenhalterung (5) ist an dem Gerätewagen (1) mehrfach verstellbar angeordnet. Die C-Bogenhalterung (5) ist mit einem Schwenklager (4) an einer horizontal verschiebbaren Horizontalführung (3) um eine horizontale Achse schwenkbar gelagert. Die Horizontalführung (3) ist an einer Säule (2) höhenverstellbar und um die senkrechte Achse der Säule (2) drehbar gelagert. Es sind vorzugsweise alle Verstelleinrichtungen des C-Bogens (6) mit Positionsmeßsensoren ausgerüstet, deren Meßwerte einer zentralen Bewegungssteuerung der Röntgendiagnostikeinrichtung zugeführt werden. Es ist vorgesehen, alle Verstellachsen wahlweise einzeln oder in der Gesamtheit durch Bremsen arretierbar auszuführen. Insbesondere ist es vorgesehen, die Rollen (20, 20') mit einer Feststellbremse auszurüsten. Vorzugsweise sind die Verstellbewegung des C-Bogens in der Halterung (Orbitalbewegung, Winkel α), die Verstellung in der Horizontalführung (3) (Horizontalverschiebung, y-Achse) und die vertikale Verstellung in der Säule (Höhenverstellung, z-Achse) elektromotorisch verstellbar ausgeführt, wobei die in den Verstellachsen angeordneten Motoren von einer zentralen Steuerung (21) gesteuert werden.

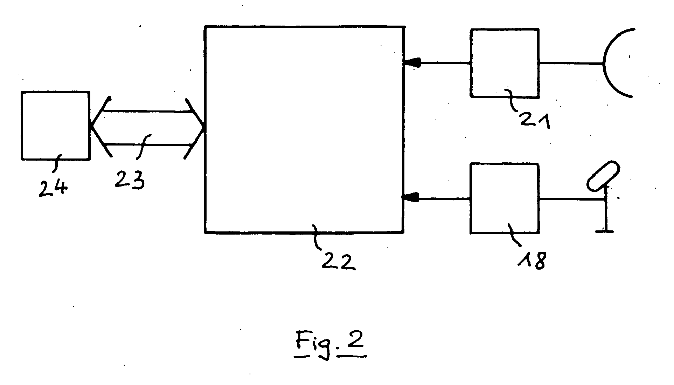

In Fig. 2 ist die Schaltung der erfindungsgemäßen Röntgendiagnostikeinrichtung mit einem Lageerfassungssystem (18) schematisch dargestellt. Ein Steuerrechner 21 steuert alle Bewegungs- und Röntgenvorgänge der Röntgendiagnostikeinrichtung und steht mit einer Recheneinheit (22) in Verbindung. Die Recheneinheit (22) enthält alle Einrichtungen zur Verarbeitung und Speicherung des kinematischen Modells der Röntgendiagnostikeinrichtung, der aufgenommenen 2D-Projektionen und der im Rahmen der Kalibrierung gewonnenen Korrekturtabellen (look-up-tables, LUT) und Einrichtungen zur Rekonstruktion eines 3D-Volumens aus 2D-Röntgenprojektionen. Des weiteren sind in der Recheneinheit (22) Module zur Durchführung von Koordinatentransformationen enthalten. An die Recheneinheit (22) ist ein Lageerfassungssystem (18) angeschlossen. Eine Datenschnittstelle (23) erlaubt den Datenaustausch zwischen der Recheneinheit (22) und einer nicht näher beschriebenen Navigationseinrichtung (24). Es ist auch vorgesehen, das Lageerfassungssystem (18) für Navigationszwecke zu verwenden und an die Datenschnittstelle (23) anzuschließen.

Für den Kalibriervorgang wird das Lageerfassungssystem (18) mit dem daran angeordneten Röntgenphantom (15) mittels eines Stativs (14) derart im Strahlengang der Röntgendiagnostikeinrichtung positioniert, daß wenigstens Teile des Röntgenphantoms vom Strahlengang erfaßt und auf dem Röntgenstrahlenempfänger (7) abgebildet werden und daß das Lageerfassungssystem eine Markenanordnung (16) erfaßt. Von dem Röntgenphantom (15) wird für wenigstens zwei verschiedene Projektionsgeometrien jeweils eine 2D-Projektionsaufnahme aufgenommen und die Lage der Markenanordnung für wenigstens eine Projektionsgeometrie ermittelt. Aus der gemessenen Lage des Röntgenphantoms (15) bezüglich der Markenanordnung (16), der bekannten Lage der Markenanordnung (16) in einem Koordinatensystem des Röntgendiagnostikeinrichtung und aus der Bestimmung der Lage der Projektionen des Röntgenphantoms (15) in den 2D-Röntgenprojektionsaufnahmen ist die Koordinatentransformation mit nur einer einzigen Lageerfassung möglich.

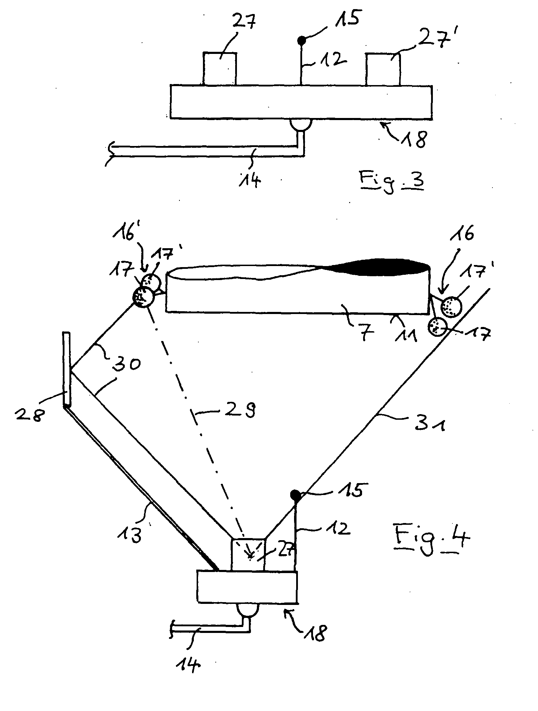

In Fig. 3 ist ein Röntgenphantom (15) schematisch dargestellt, das mit einer Halterung (12) an der Aufnahmeeinheit eines Lageerfassungssystems (18) gehalten wird. Das Röntgenphantom ist in diesem Ausführungsbeispiel als quasi punktförmige röntgenpositive Marke ausgebildet, wobei die Halterung (12) im wesentlichen röntgentransparent ist. Die Aufnahmeeinheit des Lageerfassungssystems ist an einem Stativ (14) verstellbar gelagert und weist zwei Kameras (27, 27') einer Stereokamera auf.

In Fig. 4 ist ein Röntgenphantom (15) schematisch dargestellt, das mit einer vorzugsweise röntgentransparenten Halterung (12) an einer Kamera (27) gehalten wird, wobei die Kamera (27) an einem Stativ (14) verstellbar gelagert ist. An der Kamera (27) ist weiterhin ein planer Spiegel (28) mittels einer Spiegelhalterung (13) in bekannter Orientierung zu der Kamera (27) derart gelagert, daß wenigstens einige der Marken (17, 17') der Markenanordnung (16, 16') am Röntgenstrahlenempfänger (7) von der Kamera mit einem direkten Strahl (29) und mit einem reflektierten Strahl (30) erfaßbar sind. Die Kamera (27) weist vorzugsweise einen großen Öffnungswinkel auf, der in der Darstellung der Fig. 4 durch einen Grenzstrahl (31) begrenzt ist. Das in Fig. 4 dargestellte Lageerfassungssystem (18) mit einer einzigen Kamera (27) stellt eine kostengünstige Möglichkeit dar, die Position eines Röntgenphantoms (15) in Bezug zu den Marken (17, 17') der Markenanordnung (16, 16') des Röntgenstrahlenempfängers (7) zu bestimmen, wenn kein anderes Lageerfassungssystem für die Kalibrierung der Röntgendiagnostikeinrichtung zur Verfügung steht.

Die Halterung (12) des Röntgenphantoms (15) an der Aufnahmeeinheit des Lageerfassungssystems (18) ist vorzugsweise lösbar und reproduzierbar positionierbar, beispielsweise durch Einschrauben, gehalten.

In der Anmeldung zitierte Patentdokumente:
DE 102 02 091 A1
DE 100 47 382 C2

### Bezugszeichenliste:

- 1: Gerätewagen
- 2: Säule
- 3: Horizontalführung
- 4: Schwenklager
- 5: C-Bogenhalterung
- 6: C-Bogen
- 7: Röntgenstrahlenempfänger
- 8: Röntgenstrahlenquelle
- 9: Brennfleck
- 10: Zentralstrahl
- 11: Eingangsfenster
- 12: Halterung
- 13: Spiegelhalterung
- 14: Stativ
- 15: Röntgenphantom
- 16, 16': Markenanordnung
- 17, 17': Marke
- 18: Lageerfassungssystem
- 19: Fußboden
- 20, 20': Rolle
- 21: Zentrale Steuerung
- 22: Recheneinheit
- 23: Datenschnittstelle
- 24: Navigationseinrichtung
- 26: Mittelpunkt des C-Bogens
- 27, 27': Kamera
- 28: Spiegel
- 29: Direkter Strahl
- 30: Reflektierter Strahl
- 31: Grenzstrahl

## Patentansprüche

1. Vorrichtung zur Verwendung bei einem Kalibrierverfahren für eine Röntgendiagnostikeinrichtung mit einem Röntgenphantom (15) und einem Lageerfassungssystem (18) zur Erfassung einer Markenanordnung (16, 16'),
**dadurch gekennzeichnet, daß** das Röntgenphantom (15) in einer bezüglich des Koordinatensystems des Lageerfassungssystems (18) vorbestimmten Lage und Orientierung zu und in der Nähe von der Aufnahmeeinheit des Lageerfassungssystems (18) angeordnet und an dieser mit einer Halterung (12) gehalten ist.

2. Vorrichtung nach Anspruch 1,
**dadurch gekennzeichnet, daß** das Röntgenphantom (15) an der Aufnahmeeinheit des Lageerfassungssystems (18) mit einer Halterung (12) lösbar und reproduzierbar lagegetreu gehalten ist.

3. Vorrichtung nach Anspruch 1,
**dadurch gekennzeichnet, daß** das Röntgenphantom (15) in die Aufnahmeeinheit des Lageerfassungssystems (18) integriert ist.

4. Vorrichtung nach einem der Ansprüche 1-3,
**dadurch gekennzeichnet, daß** die Aufnahmeeinheit des Lageerfassungssystems (18) eine einzige Kamera (27) und wenigstens einen ebenen Spiegel (28) im Strahlengang zwischen der Kamera (27) und der Markenanordnung (16) aufweist, wobei das Bildfeld der Kamera einen ersten Bereich aufweist, auf dem die Markenanordnung mittels eines direkten Strahls (29) und einen zweiten und vorzugsweise weitere Bereiche aufweist, in denen die Markenanordnung (16) über wenigstens einen am Spiegel (28)reflektierten Strahl (30) abgebildet wird.

5. Verfahren zur Kalibrierung einer Röntgendiagnostikeinrichtung mit einer Vorrichtung nach einem der Ansprüche 1-4,
**gekennzeichnet durch die folgenden Verfahrensschritte:**
• Positionierung der Aufnahmeeinheit des Lageerfassungssystems (18) mit dem daran angeordneten Röntgenphantom (15) im Strahlengang der Röntgendiagnostikeinrichtung in einer ersten Projektionsgeometrie derart, daß wenigstens ein Teil des Röntgenphantoms (15) auf einen Röntgenstrählenempfänger (7) mittels einer Röntgenstrahlenquelle (8) projizierbar und die Markenanordnung (16, 16') vom Lageerfassungssystem (18) erfaßbar ist
• Aufnahme der 2D-Röntgenprojektion des Röntgenphantoms (15) und Bestimmung der Lage der Markenanordnung (16, 16') mittels des Lageerfassungssystems (18) in der ersten Projektionsgeometrie
• Aufnahme einer 2D-Röntgenprojektion des Röntgenphantoms (15) in wenigstens einer zweiten Projektionsgeometrie und 3D-Rekonstruktion des Röntgenphantoms (15)
• Berechnung der Koordinatentransformation zwischen dem Koordinatensystem des Röntgenphantoms (15) und der Röntgendiagnostikeinrichtung aus der ermittelten Lage des Röntgenphantoms (15) bezüglich der Markenanordnung (16, 16') und der Lage des Projektionsbildes des Röntgenphantoms (15) im rekonstruierten Volumen bezüglich der Markenanordnung (16, 16').
